# EUROPÄISCHE PATENTSCHRIFT

(11) **EP 0 680 272 B1**
(45) Veröffentlichungstag und Bekanntmachung des Hinweises auf die Patenterteilung: **27.08.1997**
(21) Anmeldenummer: 94904638.7
(22) Anmeldetag: 07.01.1994
(51) Int. Cl.: A61B 3/107, G01B 11/24

(54) **VERFAHREN UND VORRICHTUNG ZUR BESTIMMUNG DER TOPOGRAPHIE EINER REFLEKTIERENDEN OBERFLÄCHE**
PROCESS AND DEVICE FOR DETERMINING THE TOPOGRAPHY OF A REFLECTING SURFACE
PROCEDE ET DISPOSITIF PERMETTANT DE DETERMINER LA TOPOGRAPHIE D'UNE SURFACE REFLECHISSANTE

(30) Priorität: 21.01.1993 DE 4301525; 19.03.1993 DE 4308949; 29.07.1993 DE 4225494
(43) Veröffentlichungstag der Anmeldung: 08.11.1995
(73) Patentinhaber: TECHNOMED GESELLSCHAFT FÜR MED. UND MED.-TECHN.SYSTEME MBH, 52499 Baesweiler (DE)
(72) Erfinder: JEAN, Benedikt, D-88138 Sigmarszell (DE); BENDE, Thomas, D-72116 Mössingen (DE); MATALLANA-KIELMANN, Michael, D-72070 Tübingen-Hirshau (DE)
(74) Vertreter: Castell, Klaus, Dr.-Ing.
(86) Internationale Anmeldenummer: EP9400027
(87) Internationale Veröffentlichungsnummer: WO9416611

(56) Entgegenhaltungen:
- EP-A- 0 076 866
- WO-A-91/09564
- US-A- 4 597 648
- US-A- 4 863 260
- US-A- 5 214 456

## Beschreibung

Die Erfindung betrifft ein Verfahren und eine Einrichtung zur Bestimmung der Oberflächentopographie einer Augenhornhaut nach dem Oberbegriff der Ansprüche 1 und 11.

Derartige Verfahren sind insbesondere bei der Vermessung der Augenhornhaut basierend auf einem Moiré-Musterprojektionsverfahren unter der Bezeichnung Videokeratometrie bekannt. Hierunter versteht man die Projektion der sogenannten Placidoscheibe, d.h. eines Musters aus konzentrischen, alternierend schwarzen und weißen Ringen, auf die menschliche Augenhornhaut. Die Reflexionen von der Hornhautoberfläche werden hierbei von einer Videokamera zur rechnerischen Verarbeitung aufgenommen. Von besonderem Interesse sind hierbei die Abstände oder Verformungen der Struktur des Reflexionsmusters im Vergleich mit einem Meßnormal.

Im Falle der Vermessung einer Augenhornhaut besteht das Meßnormal in einer bekannten Oberfläche, die für eine korrekte Auswertung/Bewertung der Hornhautoberfläche benötigt wird. Die Analyse der genannten Abweichungen vom Meßnormal liefert Aussagen über den Krümmungsradius der Hornhaut sowie über Abweichungen von einer sphärischen Oberfläche, wie sie bei einem Astigmatismus vorhanden sind.

Bekannte Druckschritten zu einem solchen Verfahren sind beispielsweise die US 4,978,213, US 4,863,260 und US 4,772,115.

Die Zuordnung des Reflexionsmusters zu den eingeblendeten Projektionsringen stellt sich bei diesem Verfahren häufig als schwieriges und nicht selten mit Fehlern behaftetes Unterfangen dar. Die Schwierigkeiten bei dieser Zuordnung sind besonders groß, wenn es infolge einer defekten Hornhautoberfläche zu Lücken im Reflexionsmuster eines ursprünglich in sich geschlossenen, auf die Hornhaut projizierten Rings kommt. Durch die falsche Zuordnung nicht zusammengehöriger Strukturen eines Reflexionsmusters zu einem bestimmten eingeblendeten Projektionsring ergeben sich gravierende Folgefehler, beispielsweise in Form einer falschen Bestimmung des Hornhautradius oder in Form einer falschen Bestimmung der Abweichungen der Hornhautoberflächen von der gewünschten Kugelfläche.

Außer der Einblendung eines ringförmigen Projektionsmusters ist auch die Verwendung eines gitterförmigen Linienmusters bekannt geworden. Hierdurch können jedoch die oben genannten Nachteile nicht behoben werden.

Die bislang bekannten Verfahren sind zudem äußerst empfindlich gegen Dejustierungen des Auges in der Z-Achse, d.h. in der Verbindungsachse zwischen dem Hornhautapex und der Bilderfassungseinheit.

Die Erfindung hat daher die Aufgabe, ein Verfahren zur Bestimmung der Topographie einer reflektierenden Oberfläche und insbesondere der Augenhornhaut vorzuschlagen, bei dem eine eindeutige und zuverlässige Zuordnung der Strukturen des von der Oberfläche reflektierten Reflexionsmusters zu denen des eingeblendeten Projektionsmusters gewährleistet ist.
Weiter soll eine geeignete Vorrichtung zur Durchführung des Verfahrens vorgesehen werden.
Diese Aufgabe wird ausgehend von einem Verfahren bzw. einer Vorrichtung einleitend genannter Art durch die kennzeichnenden Merkmale der Ansprüche 1 und 11 gelöst. Durch die in den Unteransprüchen genannten Maßnahmen werden zudem vorteilhafte Weiterbildungen und Verbesserungen der Erfindung möglich.

Erfindungsgemäß werden innerhalb des auf die Oberfläche projizierten Projektionsmusters wenigstens drei unterscheidbare Erkennungsmarkierungen zur Kennzeichnung der Strukturen des Projektionsmusters verwendetet, wobei wenigstens eine Erkennungsmarkierung eine Farbmarkierung ist. Diese Verwendung besonders gekennzeichneter Zonen innerhalb des Projektionsmusters liefert in Verbindung mit z.B. hellen und dunklen Zonen ein zusätzliches Zuordnungskriterium der Strukturen des von der reflektierenden Oberfläche reflektierten Bildmusters zu dem auf die Oberfläche projizierten Projektionsmuster. Selbstverständlich wird die Anzahl der Zuordnungskriterien durch die Verwendung mehrerer solcher Erkennungsmarkierungen erhöht und somit diese Zuordnung weiter verbessert. Bei Verwendung von mindestens drei Markierungen mit voneinander unterscheidbaren Farben kann auf schwarzweiß-Markierungen auch verzichtet werden.

Weiterhin können die Strukturen des Reflexionsmusters aufgrund der Farbmarkierungen problemlos dem entsprechenden Projektionsmuster zugeordnet werden. Insbesondere sind auch Überschneidungen zweier Markierungen durch das Auftreten der entsprechenden Mischfarbe erkennbar.

Denkbar wäre allerdings auch die Verwendung einer anderen Erkennungsmarkierung, beispielsweise einer Schraffur. Grundsätzlich ist nur zu beachten, daß die Erkennungsmarkierungen, im Falle einer Schraffur die Strukturen dieser Schraffur, in einem Projektionsmuster z.B. bestehend aus einheitlich durchgehenden schwarz/weiß- bzw. hell/dunkel-Markierungen, erkennbar sind.

Das erfindungsgemäße Verfahren zur Bestimmung der Topographie einer reflektierenden Oberfläche ist in vielfältigen, nahezu beliebigen technischen Anwendungsbereichen, wie z.B. bei Kugeln, verwendbar. In einer speziellen Anwendung wird dieses Verfahren zur Vermessung der Oberflächentopographie einer Augenhornhaut angewandt.

Dies wird in vorteilhafter Weise über einen von z.B. einer weißen Lichtquelle beleuchteten Projektionskörper mit transparenten, vorzugsweise ringförmig ausgebildeten Zonen in verschiedenen Farben bewerkstelligt. Es wäre jedoch ohne weiteres auch eine Anordnung von verschiedenfarbigen Lichtquellen, beispielsweise in Form eines Diodenarrays, denkbar.

In einer bevorzugten Ausgestaltung wird ein Projektionskörper in Form eines Hohlkonus oder eines Hohlellipsoiden mit transparenten Ringen, insbesondere Farbringen,in der Seitenwand verwendet. Durch die Verwendung derartiger Hohlformen, die mit ihrer konkaven Seite zum Auge hin angeordnet werden, kann der Projektionskörper mit vergleichsweise kleinem Durchmesser in Bezug auf die Z-Achse ausgeführt werden. Die Z-Achse ergibt sich, wie oben angeführt, durch die Verlängerung der Achse der Bilderfassungseinheit zur zu vermessenden Oberfläche hin.

Vorzugsweise wird ein Bilddetektor in Form einer Farbvideokamera verwendet. Es wäre jedoch ebenso die Verwendung einer schwarz/weiß-Kamera in Verbindung mit entsprechenden Farbfiltern denkbar. In diesem Fall würde die Messung der einzelnen Farbringe nacheinander mit entsprechendem Filterwechsel durchgeführt.

Vorteilhafterweise wird das Reflexionssignal von der Hornhautoberfläche integral in seiner Intensität gemessen. Dies dient zur Kompensation von Schwankungen der Umgebungsbeleuchtung, die zufällig verteilt sind und sich auf diese Weise gegenseitig aufwiegen.

Besonders empfehlenswert ist es, die Signalverarbeitung automatisch in einer prozessorgesteuerten Auswerteeinheit mit Ausgabemonitor durchzuführen. In einer besonders vorteilhaften Ausführungsform werden die Ergebnisse hierbei in Form von Isorefraktionslinien ausgegeben. Hiermit sind diejenigen Bereiche der Oberfläche gemeint, die im Verlaufe der beschriebenen Messung die gleiche Brechkraft aufweisen. Somit sind die Abweichungen vom Meßnormal direkt und ohne die Notwendigkeit einer weiteren Interpretation der Meßdaten sichtbar. Zur unmittelbaren Unterscheidung rechnerisch, beispielsweise mittels Interpolation, ermittelter Bildpunkte von gemessenen Werten kann eine unterschiedliche Darstellungsweise, beispielsweise transparent im Gegensatz zu opak, gewählt werden.

Mit einer derart automatisierten Auswerteeinheit ist zudem eine Steuerung der Projektionseinheit möglich. Dies bedeutet, daß sämtliche Parameter der Projektionseinheit mit den jeweiligen Ergebnissen der Auswerteeinheit im Hinblick auf eine größtmögliche Eindeutigkeit der Oberflächentopographie automatisch oder interaktiv optimiert werden.

Besonders wichtig bei dem genannten Verfahren ist eine gute Justage der Oberfläche, beispielsweise der Hornhautoberfläche, im Bezug auf die Bilderfassungseinheit in Z-Richtung. Die Abbildungseigenschaften der gesamten Anordnung hängen empfindlich von diesem Abstand ab. Die Justage in Richtung der Z-Achse wird vorzugsweise durch die Einblendung wenigstens zweier Zentrierungsobjekte im Projektionsmuster mit einem bestimmten Winkel zwischen ihren jeweiligen Einblendachsen durchgeführt. Der Schnittpunkt beider Einblendachsen gibt hierbei die gewünschte korrekte Z-Position wieder. Eine Verschiebung dieser Position in Z-Richtung wird bei einer derartigen Anordnung durch eine laterale Verschiebung der reflektierten Bilder der beiden Zentrierungsobjekte in Relation zueinander widergespiegelt. Selbstverständlich können auch die Zentrierungsobjekte farblich markiert sein, wobei sich bei einer Überlagerung beider Objekte vorteilhafterweise eine Mischfarbe ergeben kann.

Ein Zentrierungsobjekt kann hierbei erzeugt werden von einem Laserstrahl, der unter einem Winkel zur Z-Achse verläuft und diesen in der gewünschten Position schneidet.

Auch ohne korrekte Justage der Z-Position kann der hierdurch auftretende Fehler mittels der so ermittelten Abweichungen von der Soll-Position rechnerisch im Auswerteverfahren korrigiert werden.

Im Falle einer medizinischen Anwendung, insbesondere zur Bestimmung der Oberflächentopographie der Augenhornhautoberfläche, kann eine Meßvorrichtung, die nach dem genannten Verfahren arbeitet, direkt an ein Operationsmikroskop angekoppelt werden, wodurch der operierende Arzt direkt in die Lage versetzt ist, Hornhautbereiche mit Abweichung von der gewünschten Geometrie zu erkennen und unmittelbar entsprechend zu behandeln.

Durch die Verwendung einer hochauflösenden Kamera in Verbindung mit einer entsprechenden Zoomoptik kann diese Technik auch zur Messung mit einer Auflösung im Mikrometerbereich durchgeführt werden. Somit ist die Messung kleinster Veränderungen in der Rauhigkeit innerhalb kleinerer Oberflächensegmente der Hornhaut möglich.

Zur Herstellung eines entsprechenden Hohlellipsoiden bei der Verwendung eines statischen Projektionskörpers sind verschiedene Verfahren denkbar.

Eine Möglichkeit hierzu bietet beispielsweise die sogenannte Folientechnik. Hierbei wird zunächst eine Farbfolie mit kreisförmigen, mehrfarbigen Ringen erstellt, anschließend an einen Projektionskopf mit der Form eines Hohlellipsoiden angepaßt und schließlich eingeschweißt.

Eine weitere Möglichkeit besteht in der sogenannten Sandwichtechnik oder Multilayertechnik. Hierbei werden aus mehreren farbigen Plexiglasblöcken Ringe in entsprechender Dicke und Farbe mittels einer Drehbank oder Fräseinheit abgestochen. Diese werden auf einer Negativform fixiert und miteinander verklebt. Dieser Verbund kann auch durch schichtweises Vergießen verschiedenfarbiger Kunststoffe hergestellt werden. Anschließend wird dieser Verbund, beispielsweise auf einer Drehbank, der gewünschten Geometrie angepaßt.

In einer dritten Ausführungsform wird ein transparenter Plexiglasrohling mit feinen eingeritzten Ringen entsprechend der späteren Farbringposition versehen, der gesamte Rohling wird sodann schwarz eingefärbt. In den rillenförmig eingeritzten Ringen kann die schwarze Farbe anschließend entfernt und mit entsprechenden Transparentfarben ersetzt werden.

Ein Ausführungsbeispiel der Erfindung sowie die zu lösenden Schwierigkeiten bei der Zuordnung zwischen Projektionsmuster und Reflexionsmuster werden in der nachfolgenden Zeichnung verdeutlicht und anhand der einzelnen Figuren im folgenden näher erläutert.

Es zeigen:
- Figur 1: das Reflexionsmuster von einem aus mehreren konzentrischen Ringen bestehenden Projektionsmuster im Falle einer gesunden, d.h. sphärischer Oberfläche versehenen, Hornhaut;
- Figur 2: ein Beispiel eines vergleichbaren Reflexionsmusters im Falle einer von der sphärischen Form abweichenden Hornhaut;
- Figur 3: eine schematische Darstellung eines Aufbaus zur Durchführung des erfindungsgemäßen Verfahrens mit statischem Projektionskörper;
- Figur 4-6: die Bilder zweier Zentrierungsobjekte bei unterschiedlicher Stellung der Hornhaut in Richtung der Z-Achse bezüglich des Bilddetektors.

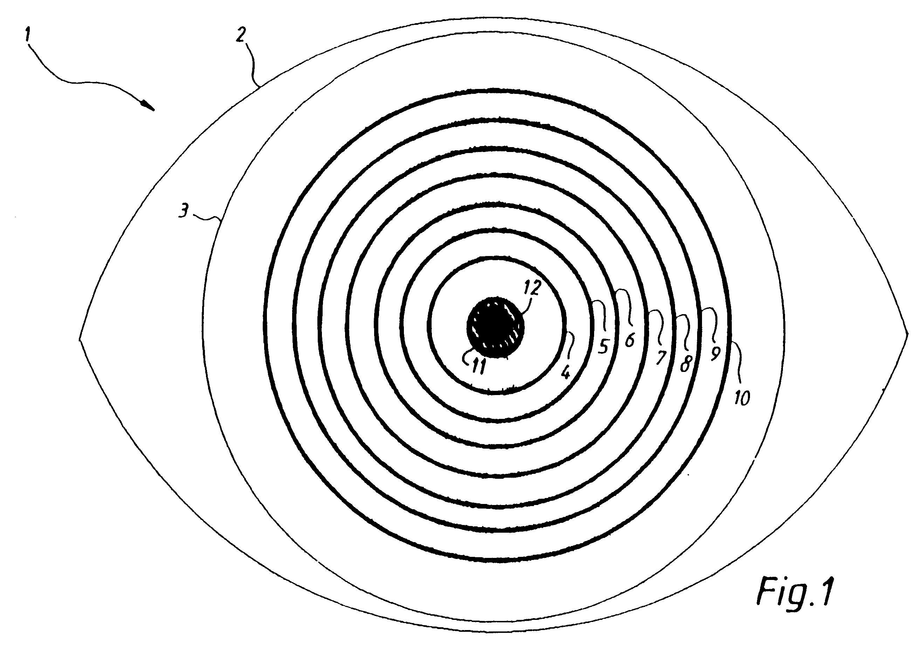

In Figur 1 ist schematisch das Bild eines Auges 1 mit mandelförmigem Umriß 2 dargestellt, wie es von einem Bilderfassungssystem der oben beschriebenen Art erfaßt wird. Im Innern der Umrißlinie 2 ist der Umriß der Hornhaut 3 eingezeichnet. Konzentrisch zur Z-Achse (Kamera-Hornhautapex) sind verschiedene Reflexionsringe 4 bis 10 gezeigt. Im Innern dieser Ringe befinden sich zwei Zentrierungsobjekte, auf die weiter unten näher eingegangen wird. Die Ringe 4 bis 10 entsprechen in ihrem Abstand sowie in ihrer konzentrischen und kreisförmigen Anordnung dem reflektierten Bild von einer gesunden Hornhaut mit einer sphärischen Oberfläche.

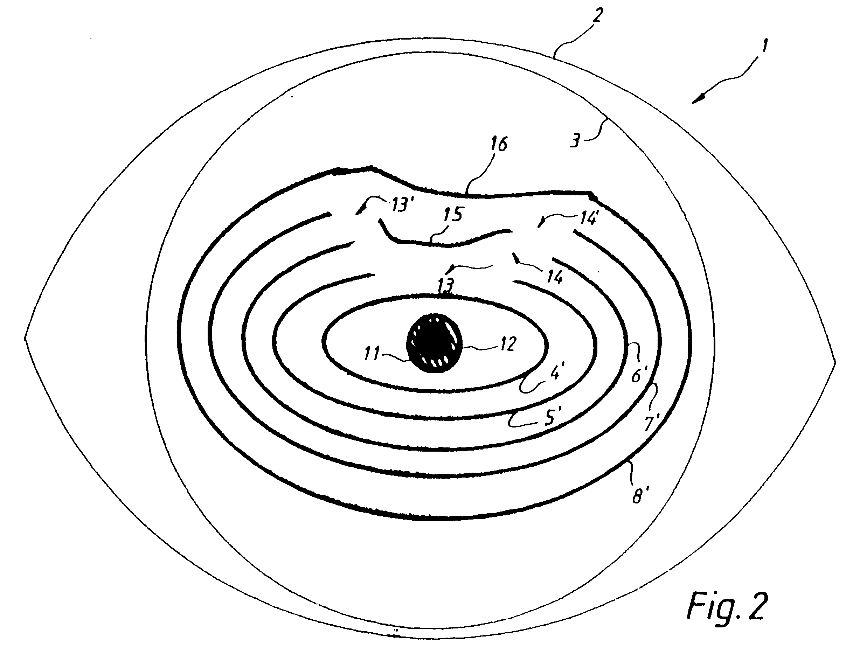

Im Gegensatz hierzu zeigt Figur 2 das entsprechende Bild bei deformierter Hornhaut, d.h. im Falle eines vorliegenden Astigmatismus. Die Strukturen 4' bis 8' stellen ebenfalls ein Reflexionsmuster von absolut konzentrischen und kreisförmigen Projektionsringen dar. Ihr Bild wird durch eine nicht sphärische Hornhautoberfläche deformiert. Teilweise weisen die Bilder 5',6' der entsprechenden und ursprünglichen geschlossenen Projektionsringe sogar Lücken 13, 14 bzw. 13', 14' auf, während andere Strukturen 7', 8' starke Einbuchtungen 15,16zeigen. Bei einer normalen schwarz/weiß-Aufnahme, wie sie anders in der Zeichnung nicht darstellbar ist, wäre nun der Bereich 15 der Struktur 7' nicht eindeutig einem entsprechenden Projektionsring zuzuordnen. Dieser Bereich 15 könnte den Projektionsringen zuzuordnen sein, die bei gesunder Hornhaut (s.Figur 1) die Reflexionsringe 5, 6 oder 7 ergeben. Entsprechend der Unsicherheit bei dieser Zuordnung ist die aus der Interpretation dieser Daten resultierende Oberflächentopographie unweigerlich mit Fehlern behaftet.

Erfindungsgemäß werden jedoch die Ringe 4 bis 10 bzw. die Strukturen 4' bis 8' durch eine entsprechende Farbgebung des Projektionsmusters eingefärbt. Somit ist auch im Falle eines stark deformierten Reflexionsbildes (s. Figur 2) ein eindeutige Zuordnung möglich.

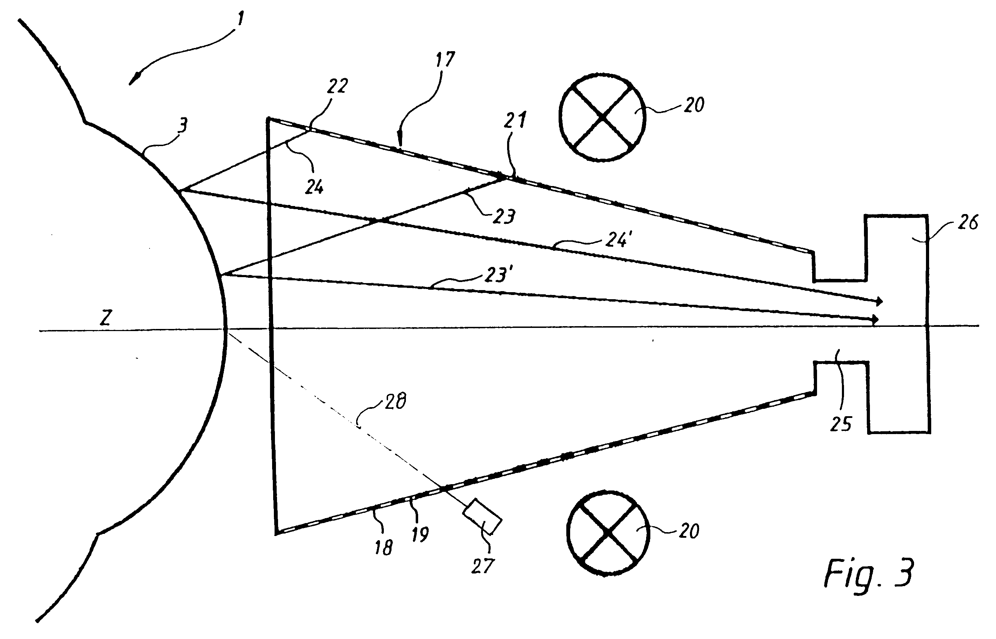

Figur 3 zeigt eine schematische Schnittdarstellung eines Aufbaus, wie er für die Durchführung des erfindungsgemäßen Meßverfahrens verwendet werden kann. Vor dem Auge 1 mit der gewölbten Hornhaut 3 befindet sich ein Projektionskörper 17.

Der Projektionskörper 17 besteht aus einem konusförmigen Hohlkörper, dessen Seitenwand 18 mit ringförmigen, transparenten und verschieden eingefärbten Durchlässen 19 versehen ist. Von außen wird der konusförmige Projektionskörper 17 durch eine ringförmige Neonröhre 20 beleuchtet. Anhand zweier von verschieden eingefärbten ringförmigen Durchlässen 21,22 ausgehenden und durch Linien 23,34 symbolisierten Lichtstrahlen wird verdeutlicht, wie die Ringstrukturen auf die Hornhaut projiziert werden. Die an der Hornhaut 3 reflektierten Strahlen 23' und 24' laufen durch eine Lochblende 25 am schmaleren Ende des konusförmigen Projektionskörper 17 und werden in einem Bilddetektor 26 abgebildet.

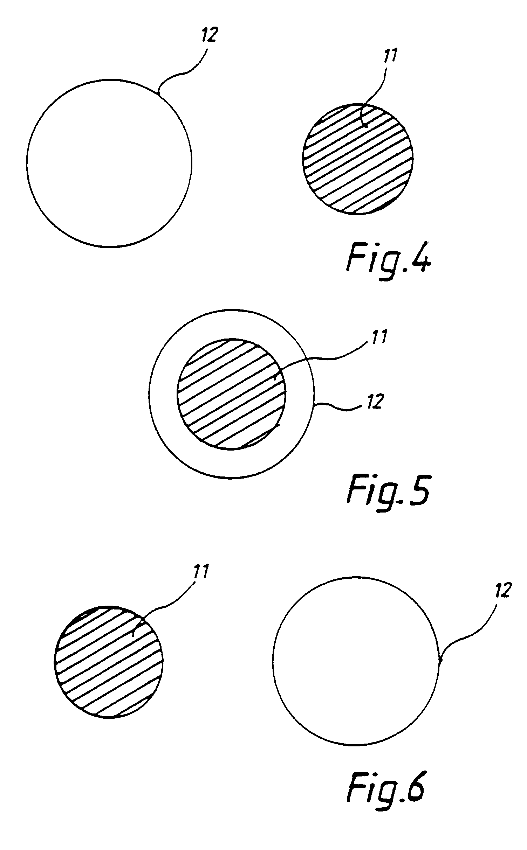

Die Zentrierungsobjekte 11, 12, die zur Erzeugung der Bilder in den Figuren 4 bis 6 unter einem bestimmten Winkel zwischen ihren Einblendachsen in einer Ebene, in der auch die Z-Achse liegt, eingeblendet werden, weisen entsprechend der Position der Hornhaut auf der Z-Achse bezüglich des Schnittpunktes der Einblendachsen eine unterschiedliche Orientierung zueinander auf. In den Figuren 4 bis 6 werden die Bilder der Zentrierungsobjekte gezeigt, wobei sich die Hornhautoberfläche 3 einmal vor diesem Schnittpunkt, einmal genau im Schnittpunkt und einmal hinter dem Schnittpunkt befindet. Durch die Anordnung der Bilder dieser Zentrierungsobjekte 11,12 kann die Z-Position der Hornhautoberfläche 3 ermittelt und für die Auswertung verwendet werden.

In Figur 3 ist zu erkennen, daß eine Einblendachse von einem Laserstrahl 28, der von einer Lasereinheit 27 ausgeht, gebildet wird. Der Laserstrahl 28 bildet z.B. auf der Hornhaut das Zentrierungsobjekt 11 ab. Die Lasereinheit 27 kann hierzu außerhalb des Projektionskörpers angeordnet sein. Hierbei hat der Projektionskörper in seiner Wandung eine kleine Öffnung für den Durchtritt des Laserstrahls 28, der dann die Z-Achse im gewünschten Punkt schneidet. Nach der Justage in Z-Richtung kann dann die Lasereinheit abgeschaltet werden.

## Patentansprüche

1. Verfahren zur Bestimmung der Topographie einer Augenhornhaut basierend auf einem Moiré-Musterprojektionsverfahren, wobei ein Projektionsmuster auf der Oberfläche der Augenhornhaut gespiegelt wird und ein von den zugehörigen Reflexionen auf der Oberfläche gebildetes Reflexionsmuster erfaßt und ausgewertet wird, wobei zur verbesserten Zuordnung der Strukturen (4 bis 10) des Reflexionsmusters zu denen des Projektionsmusters wenigstens drei unterscheidbare Erkennungsmarkierungen zur Kennzeichnung der Strukturen des Projektionsmusters verwendet werden, dadurch gekennzeichnet, daß wenigstens eine Erkennungsmarkierung eine Farbmarkierung mit einer von Schwarz und Weiß unterschiedlichen Farbe ist

2. Verfahren nach Anspruch 1, dadurch gekennzeichnet, daß als wenigstens eine Erkennungsmarkierung eine Schraffur der entsprechenden Zone des Projektionsmusters verwendet wird.

3. Verfahren nach einem der vorhergehenden Ansprüche, dadurch gekennzeichnet, daR das Projektionsmuster über einen Projektionskörper (17) mit transparenten, wenigstens teilweise mit wenigstens einer Farbe eingefärbten Lichtdurchlässen (19,21,22) erzeugt wird.

4. Verfahren nach Anspruch 3, dadurch gekennzeichnet, daß als Projektionskörper (17) ein Hohlkonus oder ein Hohlellipsoid mit transparenten Ringen (19,21,22) in seiner Seitenwand (18) verwendet wird.

5. Verfahren nach einem der vorhergehenden Ansprüche, dadurch gekennzeichnet, daß das auf der Oberfläche gebildete Reflexionsmuster von einer Farbvideokamera (26) erfaßt wird.

6. Verfahren nach einem der Ansprüche 1 bis 4, dadurch gekennzeichnet, daß das auf der Oberfläche gebildete Reflexionsmuster von einer schwarz/weiß-Kamera in Verbindung mit einem oder mehreren entsprechenden Farbfiltern erfaßt wird.

7. Verfahren nach einem der vorhergehenden Ansprüche, dadurch gekennzeichnet, daß eine integrale Intensitätsmessung der Reflexionsstrukturen zur Kompensation von Schwankungen der Umgebungsbeleuchtung durchgeführt wird.

8. Verfahren nach einem der Ansprüche 5 oder 6, dadurch gekennzeichnet, daß die Signalverarbeitung der Ausgangssignale der Kamera (26) automatisch in einer prozessorgesteuerten Auswerteeinheit durchgeführt wird, wobei auf einem Ausgabemonitor Isorefraktionslinien der vermessenen Oberfläche dargestellt werden.

9. Verfahren nach Anspruch 8, dadurch gekennzeichnet, daß mit Hilfe der Auswerteeinheit eine Steuerung zur Optimierung des Projektionsmusters im Hinblick auf eine eindeutige Oberflächentopometrie durchgeführt wird.

10. Verfahren nach einem der Ansprüche 5 bis 9, dadurch gekennzeichnet, daß die Position der Oberfläche entlang einer Z-Achse, d.h. entlang der Verlängerung der Achse der Kamera, durch die Einblendung wenigstens zweier Zentrierungsobjekte (11,12) mit einem Winkel zwischen ihren Einblendachsen erfaßt wird, wobei der Schnittpunkt beider Einblendachsen die korrekte Z-Stellung angibt.

11. Vorrichtung zur Bestimmung der Topographie einer Augenhornhaut basierend auf einem Moiré-Musterprojektionsverfahren, mit einer Projektionseinheit zur Projektion eines Projektionsmusters auf die Augenhornhaut, einer Bilderfassungseinheit zur Erfassung des Spiegelbildes des Projektionsmusters und einer Auswerteeinheit zum Vergleich des Spiegelbildes mit einem Meßnormal, wobei die Projektionseinheit für eine Markierung des Projektionsmusters mit mindestens drei unterscheidbaren Erkennungsmarkierungen ausgebildet ist, dadurch gekennzeichnet, daß wenigstens eine Erkennungsmarkierung eine Farbmarkierung mit einer von Schwarz und Weiß unterschiedlichen Farbe ist.

12. Vorrichtung nach Anspruch 11, dadurch gekennzeichnet, daß die Projektionseinheit einen als Hohlkonus oder Hohlellipsoid ausgebildeten Projektionskörper (17) aufweist mit transparenten Ringen in der Wand und einer außen angeordneten Beleuchtungseinheit (20).

13. Vorrichtung nach Anspruch 11 und 12, dadurch gekennzeichnet, daß der Projektionseinheit eine Lasereinheit (27) zur Einblendung eines Zentrierobjektes (11) zugeordnet ist.

14. Vorrichtung nach Anspruch 13, dadurch gekennzeichnet, daß der Projektionskörper (17) in seiner Wand eine Öffnung für den Durchtritt der Laserstrahlen (28) der Lasereinheit (27) aufweist.

## Claims

1. Process for determining the topography of a cornea based on a moiré pattern projection process, wherein a projection pattern is reflected on the surface of the cornea and a reflection pattern formed by the corresponding reflections on the surface is detected and evaluated, wherein at least three distinguishable identification markings for identification of the structures of the projection pattern are used for improved allocation of the structures (4 to 10) of the reflection pattern to those of the projection pattern, characterised in that at least one identification marking is a colour marking with a colour other than black and white.

2. Process according to Claim 1, characterised in that hatching of the corresponding zone of the projection pattern is used as at least one identification marking.

3. Process according to one of the preceding claims, characterised in that the projection pattern is generated via a projection body (17) with transparent light passages (19, 21, 22) at least partially coloured with at least one colour.

4. Process according to Claim 3, characterised in that a hollow cone or a hollow ellipsoid with transparent rings (19, 21, 22) in its side wall (18) is used as projection body (17).

5. Process according to one of the preceding claims, characterised in that the reflection pattern formed on the surface is detected by a colour video camera (26).

6. Process according to one of Claims 1 to 4, characterised in that the reflection pattern formed on the surface is detected by a black/white camera in conjunction with one or more appropriate colour filters.

7. Process according to one of the preceding claims, characterised in that an integral intensity measurement of the reflection structures is carried out for compensation of fluctuations in the ambient lighting.

8. Process according to one of more of Claims 5 or 6, characterised in that the signal processing of the output signals of the camera (26) is carried out automatically in a processor-controlled evaluation unit, whereby iso-refraction lines of the measured surface are displayed on an output monitor.

9. Process according to Claim 8, characterised in that a control is carried out by means of the evaluation unit for optimisation of the projection pattern with respect to a well defined surface topometry.

10. Process according to one of Claims 5 to 9, characterised in that the position of the surface along a Z-axis, i.e. along the extension of the axis of the camera, is determined by the fading in of at least two centring objects (11, 12) with an angle between their fade-in axes, whereby intersection of the two fade-in axes indicates the correct Z-position.

11. Device for determining the topography of a cornea based on a moiré pattern projection process, with a projection unit for projecting a projection pattern onto the cornea, an image acquisition unit for capturing the mirror image of the projection pattern and an evaluation unit for comparing the mirror image with a measurement standard, whereby the projection unit is constructed with at least three distinguishable identification markings for marking the projection pattern, characterised in that at least one identification marking is a colour marking with a colour other than black and white.

12. Device according to Claim 11, characterised in that the projection unit has a projection body (17) constructed as a hollow cone or hollow ellipsoid with transparent rings in the wall and an externally disposed lighting unit (20).

13. Device according to Claim 11 and 12, characterised in that a laser unit (27) is allocated to the projection unit for fade-in of a centring object (11).

14. Device according to Claim 13, characterised in that the projection body (17) has an opening in its wall for passage of the laser beams (28) of the laser unit (27).

## Revendications

1. Procédé destiné à la détermination de la topographie d'une cornée, basé sur une méthode de moirage de projection de modèle, selon lequel un modèle de projection est reflété sur la surface de la cornée et un modèle de réflexion formé sur la surface par des réflexions associées est enregistré et analysé alors qu'on utilise au moins trois marquages différents pour l'identification des structures du modèle de projection de manière à mieux reconnaître les structures (4 à 10) du modèle de réflexion par rapport à celles du modèle de projection, caractérisé en ce qu'au moins un marquage d'identification est une marque d'une couleur autre que le noir et le blanc.

2. Procédé selon la revendication 1 caractérisé en ce qu'on utilise une hachure de la zone correspondante du modèle de projection comme au moins une marque d'identification.

3. Procédé selon l'une des revendications précédentes caractérisé en ce que le modèle de projection est généré par l'intermédiaire d'un corps de projection (17) présentant des zones perméables à la lumière (19,21,22) transparentes et teintées au moins en partie avec au moins une couleur.

4. Procédé selon la revendication 3 caractérisé en ce qu'on utilise comme corps de projection (17) un cône creux ou un ellipsoïde creux avec des anneaux transparents (19,21,22) sur sa paroi latérale (18).

5. Procédé selon l'une des revendications précédentes caractérisé en ce que le modèle de réflexion formé sur la surface est enregistré par une caméra vidéo couleur (26).

6. Procédé selon l'une de revendications 1 à 4 caractérisé en ce que le modèle de réflexion formé sur la surface est enregistré par une caméra noir et blanc reliée avec un ou plusieurs écrans colorés appropriés.

7. Procédé selon l'une des revendications précédentes caractérisé en ce qu'on procède à une mesure intégrale de l'intensité des structures de réflexion afin de compenser les fluctuations de l'éclairage ambiant.

8. Procédé selon l'une revendications 5 ou 6 caractérisé en ce que le traitement des signaux de sortie de la caméra (26) s'effectue automatiquement dans une unité d'analyse commandée par processeur, des lignes d'isoréfraction de la surface mesurée étant représentées sur un moniteur de sortie.

9. Procédé selon la revendication 8 caractérisé en ce qu'on procède à une commande à l'aide de l'unité d'analyse de manière à optimaliser le modèle de projection et obtenir une topométrie de surface non équivoque.

10. Procédé selon l'une des revendications 5 à 9 caractérisé en ce qu'on enregistre la position de la surface le long d'un axe Z, c'est à dire le long du prolongement de l'axe de la caméra, par insertion d'au moins deux objets de centrage (11,12) présentant un angle entre leurs axes d'insertion, le point d'intersection de ces deux axes donnant la position Z correcte.

11. Dispositif destiné à la détermination de la topographie d'une cornée sur la base d'une méthode de moirage de projection de modèle, avec une unité de projection pour la projection d'un modèle de projection sur la cornée, une unité d'enregistrement de l'image pour enregistrer l'image réfléchie du modèle de projection et une unité d'analyse pour comparer l'image réfléchie avec un étalon de mesure, la structure de l'unité de projection permettant un marquage du modèle de projection avec au moins trois marques d'identification différentes, caractérisé en ce qu'au moins un marquage d'identification est une marque d'une couleur autre que le noir et le blanc.

12. Dispositif selon la revendication 11 caractérisé en ce que l'unité de projection présente un corps de projection ayant la forme d'un cône creux ou d'un ellipsoïde creux dont la paroi est pourvue d'anneaux transparents et une unité d'éclairage (20) externe.

13. Dispositif selon les revendications 11 et 12 caractérisé en ce qu'on a adjoint à l'unité de projection une unité laser (27) destinée à l'insertion d'un objet de centrage (11).

14. Dispositif selon la revendication 13 caractérisé en ce que la paroi du corps de projection (17) présente une ouverture permettant le passage des rayons laser (28) de l'unité laser (27).
